# EUROPEAN PATENT APPLICATION

(11) **EP 4 471 123 A1**
(43) Date of publication of application: **04.12.2024**
(21) Application number: 22923646.8
(22) Date of filing: 29.12.2022
(51) Int. Cl.: C12N 1/21, C12N 15/63, C12N 15/77, C12P 13/08, C12R 1/15

(54) **METHOD FOR CONSTRUCTING THREONINE-PRODUCING ENGINEERED BACTERIUM**

(30) Priority: 26.01.2022 CN 202210096134
(71) Applicant: Langfang Meihua Biotechnology Development Co., Ltd, Langfang City, Hebei 065001 (CN)
(72) Inventor: KANG, Pei, Langfang City, Hebei 065001 (CN); GONG, Weibo, Langfang City, Hebei 065001 (CN); HE, Jun, Langfang City, Hebei 065001 (CN); LI, Yan, Langfang City, Hebei 065001 (CN)
(74) Representative: CH Kilger Anwaltspartnerschaft mbB
(86) International application number: PCT/CN2022/143105
(87) International publication number: WO 2023/142861

(57) **Abstract**

The present invention provides a method for constructing a threonine-producing engineered bacterium. According to the present invention, a 2-methylcitrate synthase 1-inactivated strain (Corynebacterium) is applied to the production of threonine, and the production of threonine produced by the 2-methylcitrate synthase 1-inactivated strain is increased by about 42% compared with that produced by an unengineered strain. When the application of the 2-methylcitrate synthase 1-inactivated strain is further combined with enhanced expression of at least one of aspartate aminotransferase, aspartate kinase, homoserine dehydrogenase, threonine synthase, NAD kinase, fructose-1,6-bisphosphatase 2 and the like in the threonine synthesis pathway, the production of threonine is improved. The method provides a new way for large-scale production of threonine and has high application value.

## Description

### Technical Field

The present invention belongs to the technical field of microbial engineering, and specifically relates to a method for constructing a threonine-producing engineered bacterium.

### Background Art

L-Threonine has a chemical name of β-hydroxy-α-aminobutyric acid, a molecular formula of C₄H₉NO₃, and a relative molecular mass of 119.12. L-threonine is an essential amino acid. Threonine is mainly used in medicine, chemical reagents, food fortifiers, feed additives, etc.

In Corynebacterium glutamicum, the production of threonine from oxaloacetate requires a five-step catalytic reaction which involves aspartate kinase (encoded by lysC), aspartate semialdehyde dehydrogenase (encoded by asd), homoserine dehydrogenase (encoded by horn), homoserine kinase (encoded by thrB) and threonine synthase (encoded by thrC). Hermann Sahm et al. have been committed to the development of high threonine-producing Corynebacterium glutamicum strains and have made some breakthroughs, obtaining the feedback-resistant homoserine dehydrogenase (Reinscheid D J, Eikmanns B J, Sahm H. Analysis of a Corynebacterium glutamicum horn gene coding for a feedback-resistant homoserine dehydrogenase.[J]. Journal of Bacteriology, 1991, 173(10):3228-3230) andaspartate kinase (Eikmanns B J, Eggeling L, Sahm H. Molecular aspects of lysine, threonine, and isoleucine biosynthesis in Corynebacterium glutamicum.[J]. Antonie Van Leeuwenhoek, 1993, 64(2):145-163). Following Hermann Sahm, Lothar Eggling conducted further exploration in this field by attenuating the coding gene glyA in the threonine utilization pathway and overexpressing the threonine export protein ThrE so that the production of threonine is increased from 49 mM to 67 mM (Simic P, Willuhn J, Sahm H, et al. Identification of glyA (Encoding Serine Hydroxymethyltransferase) and Its Use Together with the Exporter ThrE To Increase 1-Threonine Accumulation by Corynebacterium glutamicum[J]. Applied and Environmental Microbiology, 2002, 68(7):3321-3327).

Current reports on using Corynebacterium glutamicum to produce threonine mainly focus on its synthesis pathway, and there are few reports on precursor supply and other aspects. Moreover, the present reports have only conducted preliminary research on the threonine synthesis pathway and have not yet formed a system.

### Summary of the Invention

The objective of the present invention is to improve the ability of a strain to produce threonine by inactivating 2-methylcitrate synthase 1, thereby providing a method for constructing an engineered bacterium that produces threonine (L-threonine).

In order to achieve the objective of the present invention, in a first aspect, the present invention provides a modified Corynebacterium microorganism, wherein the activity of 2-methylcitrate synthase 1 of the microorganism is reduced or lost compared to an unmodified microorganism, and the microorganism has an enhanced threonine-producing ability compared to an unmodified microorganism. Preferably, 2-methylcitrate synthase 1 has a reference sequence number of WP_011013823.1 on NCBI, or an amino acid sequence with 90% similarity thereto.

Further, the reduction in or loss of 2-methylcitrate synthase 1 activity in the microorganism is achieved by reducing the expression of a gene encoding 2-methylcitrate synthase 1 or knocking out an endogenous gene encoding 2-methylcitrate synthase 1.

Mutagenesis, site-directed mutation or homologous recombination can be used to reduce the expression of the gene encoding 2-methylcitrate synthase 1 or knock out the endogenous gene encoding 2-methylcitrate synthase 1.

Further, the microorganism has enhanced activity of an enzyme involved in the threonine synthesis pathway in vivo compared to the unmodified microorganism;
wherein the enzyme involved in the threonine synthesis pathway is selected from at least one of aspartate aminotransferase, aspartate kinase, homoserine dehydrogenase, threonine synthase, NAD kinase, and fructose-1,6-bisphosphatase 2; preferably, they have reference sequence numbers of WP_011013497.1, WP_003855724.1, WP_003854900.1, WP_011014964.1, NP_600631.1, and WP_003856830.1 on NCBI respectively, or amino acid sequences with 90% similarity to the above reference sequences.

Preferably, the microorganism is any of the following (1) to (5):
(1) a microorganism with reduced or lost activity of 2-methylcitrate synthase 1 and enhanced activity of aspartate kinase and/or homoserine dehydrogenase;
(2) a microorganism with reduced or lost activity of 2-methylcitrate synthase 1 and enhanced activity of aspartate kinase, homoserine dehydrogenase and/or threonine synthase;
(3) a microorganism with reduced or lost activity of 2-methylcitrate synthase 1 and enhanced activity of aspartate kinase, homoserine dehydrogenase and/or NAD kinase;
(4) a microorganism with reduced or lost activity of 2-methylcitrate synthase 1 and enhanced activity of aspartate kinase, homoserine dehydrogenase, NAD kinase and/or fructose-1,6-bisphosphatase 2;
(5) a microorganism with reduced or lost activity of 2-methylcitrate synthase 1 and enhanced activity of aspartate aminotransferase, aspartate kinase, homoserine dehydrogenase, threonine synthase, NAD kinase and fructose-1,6-bisphosphatase 2.

The enhancement of the activity of the enzymes involved in the threonine synthesis pathway in the microorganism is achieved by any one selected from the following 1) to 6), or an optional combination thereof:
1) enhancement by introduction of a plasmid having the gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme;
6) enhancement by altering the nucleotide sequence encoding the enzyme.

Preferably, the microorganism of Corynebacterium used in the present invention is Corynebacterium glutamicum. Corynebacterium glutamatum includes ATCC13032, ATCC13870, ATCC13869, ATCC21799, ATCC21831, ATCC14067, ATCC13287, etc. (see NCBI Corunebacterium glutamicum evolutionary tree https://www.ncbi.nlm.nih.gov/genome/469), more preferably Corynebacterium glutamatum ATCC 13032.

In a second aspect, the present invention provides a method for constructing an engineered threonine producing bacterium, the method comprises:
A. attenuating the gene encoding 2-methylcitrate synthase 1 in amino acid producing Corynebacterium to obtain a gene-attenuated strain; the attenuating includes knocking out or reducing the expression of the gene encoding 2-methylcitrate synthase 1; and/or
B. enhancing an enzyme involved in threonine synthesis pathway in the gene-attenuated strain in step A to obtain a strain with enhanced enzyme activity;
the way for the enhancement is selected from the following 1) to 5), or an optional combination thereof:
1) enhancement by introduction of a plasmid having the gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme.

In a third aspect, the present invention provides a method for producing threonine, the method comprises the steps of:
a) cultivating the modified Corynebacterium microorganism to obtain a culture of the microorganism;
b) collecting threonine from the culture obtained in step a).

In a fourth aspect, the present invention provides use of knocking out or reducing expression of the gene encoding 2-methylcitrate synthase 1 in the fermentative production of threonine or improving its fermentative production.

Further, the fermentative production of threonine is increased by the inactivation of 2-methylcitrate synthase 1 in *Corynebacterium* with amino acid-producing ability.

Preferably, the Corynebacterium used in the present invention is Corynebacterium glutamicum. Corynebacterium glutamatum includes ATCC13032, ATCC13870, ATCC13869, ATCC21799, ATCC21831, ATCC14067, ATCC13287, etc. (see NCBI Corunebacterium glutamicum evolutionary tree https://www.ncbi.nlm.nih.gov/genome/469), more preferably Corynebacterium glutamatum ATCC 13032.

In a fifth aspect, the present invention provides use of the modified Corynebacterium microorganism or a threonine-producing engineered bacterium constructed according to the above method in the fermentative production of threonine or increasing its fermentative production.

The above-mentioned modification methods of related strains, including gene enhancement and attenuation, are all modification methods known to those skilled in the art, see Man Zaiwei. Systemic pathway engineering modification of Corynebacterium crenatum to produce L-arginine with high yield [D]. Jiangnan University, 2016; Cui Yi. Metabolic engineering modification of Corynebacterium glutamicum to produce L-leucine [D]. Tianjin University of Science and Technology.; Xu Guodong. Construction of L-isoleucine producing strain and optimization of fermentation conditions. Tianjin University of Science and Technology, 2015.

Through the above technical solutions, the present invention has at least the following advantages and beneficial effects:
the present invention enhances the supply of oxaloacetate, a precursor for threonine synthesis, by inactivating 2-methylcitrate synthase 1, thereby improving the ability of the strain to produce threonine. According to the present invention, a 2-methylcitrate synthase 1-inactivated strain (Corynebacterium, such as Corynebacterium glutamicum) is applied to the production of threonine, and the threonine production in the 2-methylcitrate synthase 1-inactivated strain is increased by about 60% compared with unengineered strain. When the application of the 2-methylcitrate synthase 1-inactivated strain is further combined with enhanced expression of at least one of aspartate aminotransferase, aspartate kinase, homoserine dehydrogenase, threonine synthase, NAD kinase, fructose-1,6-bisphosphatase 2 and the like in the threonine synthesis pathway, the production of threonine is also improved. The method provides a new way for large-scale production of threonine and has high application value.

### Detailed Description of Embodiments

The present invention improves threonine production of a strain (such as Corynebacterium glutamicum) by inactivating 2-methylcitrate synthase 1 (prpC1).

2-methylcitrate synthase 1 is not directly involved in the threonine synthesis pathway, and there is no report on inactivation of 2-methylcitrate synthase 1 can increase the downstream products of threonine currently. To this end, the present invention first uses Corynebacterium glutamicum ATCC 13032 as an original strain to construct a 2-methylcitrate synthase 1-inactivated strain. The threonine yield of the obtained bacterium is as low as 0.2 g/L, which is inconsistent with expectations. We speculated the phenomenon is because that during threonine synthesis, aspartate kinase and homoserine dehydrogenase in the threonine synthesis pathway are strictly regulated by the intracellular threonine concentration. Therefore, in order to modify the strain to produce threonine, we first unlocked its synthesis pathway, mainly including the obtainment of feedback-resistant aspartate kinase and homoserine dehydrogenase and enhancement of aspartate kinase and homoserine dehydrogenase expression. The modified bacterium SMCT077 was obtained and had preliminary threonine synthesis ability with a threonine production of 2.4 g/L. Further inactivation of prpC1 improved the threonine production to 3.3 g/L. It can be seen that although the inactivation of 2-methylcitrate synthase 1 is beneficial to the production of threonine, when the inactivation of 2-methylcitrate synthase 1 is combined with other sites involved in threonine synthesis in the threonine-producing strain, the threonine-producing ability of the strain will be further improved.

To further verify that the increase in threonine production is due to the inactivation of 2-methylcitrate synthase 1, a series of strains were obtained by inactivating prpC1 in the strains that has further enhanced expression of at least one of aspartate aminotransferase, threonine synthase, NAD kinase and fructose-1,6-bisphosphatase on the basis of the SMCT077 strain, and the strains with inactivated prpC1 increased threonine production by 42%.

Expression enhancement during the modification process includes methods such as promoter replacement, change of ribosome binding sites, increase in copy number, change of amino acid sequence to increase activity and plasmid overexpression, and inactivation includes reduction in expression activity and inactivity, and the above methods are all well known to researchers in the art. The above methods cannot be exhaustive through examples, and the specific embodiments only use enhancement by promoter as a representative for illustration; In addition, the present invention only lists some of the modification combinations. The fact that all combinations of the above-mentioned sites can increase the production of threonine is merely exemplified herein and is not intended to be exhaustive.

### The present invention adopts the following technical solutions:

One of the technical solutions of the present invention provides a method for producing threonine using Corynebacterium in which 2-methylcitrate synthase 1 is inactivated.

A second technical solution of the present invention provides a method for producing threonine by inactivating 2-methylcitrate synthase 1 and enhancing the expression of at least one of aspartate aminotransferase, aspartate kinase, homoserine dehydrogenase, threonine synthase, NAD kinase, and fructose-1,6-bisphosphatase 2.

A third technical solution of the present invention provides a method for producing threonine by obtainment of feedback-resistant aspartate kinase and homoserine dehydrogenase , enhancement of their expression and 2-methylcitrate synthase 1 inactivation.

A fourth technical solution of the present invention provides a method for producing threonine by inactivating 2-methylcitrate synthase 1 and enhancing the expression of aspartate kinase, homoserine dehydrogenase and threonine synthase.

A fifth technical solution of the present invention provides a method for producing threonine by inactivating 2-methylcitrate synthase 1 and enhancing the expression of aspartate kinase, homoserine dehydrogenase and NAD kinase.

A sixth technical solution of the present invention provides a method for producing threonine by inactivating 2-methylcitrate synthase 1 and enhancing the expression of aspartate kinase, homoserine dehydrogenase, NAD kinase, and fructose-1,6-bisphosphatase 2.

A seventh technical solution of the present invention provides a method for producing threonine by inactivating 2-methylcitrate synthase 1 and enhancing the expression of aspartate aminotransferase, aspartate kinase, homoserine dehydrogenase, threonine synthase, NAD kinase, and fructose-1,6-bisphosphatase.

The above-mentioned strain is Corynebacterium, preferably Corynebacterium glutamicum, and most preferably Corynebacterium glutamicum ATCC 13032.

The proteins involved in the present invention and genes encoding the proteins are as follows:
2-methylcitrate synthase 1, coding gene name prpC1, NCBI number: cg0798, Cgl0696, NCgl0666.
aspartate aminotransferase, coding gene name aspB, NCBI number: cg0294, Cgl0240, NCgl0237.
aspartate kinase, coding gene name lysC, NCBI number: cg0306, Cgl0251, NCgl0247.
homoserine dehydrogenase, coding gene name horn, NCBI number: cg1337, Cgl1183, NCgl1136.
threonine synthase, coding gene name thrC, NCBI number: cg2437, Cgl2220, NCgl2139.
NAD kinase, coding gene name ppnK, NCBI number: cg1601, Cgl1413, NCgl1358.
fructose-1,6-bisphosphatase 2, coding gene name fbp/glpX, NCBI number: cg1157, Cgl1019, Ncgl0976.

The following examples are intended to illustrate the present invention but are not intended to limit the scope of the present invention. Unless otherwise specified, the examples are based on conventional experimental conditions, such as Sambrook J & Russell DW, Molecular Cloning: a Laboratory Manual, 2001, or the conditions recommended by the manufacturer's instructions.

The experimental materials used in the following examples are as follows:

| Reagents | Manufacturer |
|---|---|
| TransStart^{®} FastPfu DNA Polymerase | Beijing TransGen Biotech Co., Ltd. |
| Trans15K DNA Marker | Beijing TransGen Biotech Co., Ltd. |
| Trans1-T1 Phage Resistant Chemically Competent Cell | Beijing TransGen Biotech Co., Ltd. |
| BamHI | Thermo Fisher Scientific (China) Co., Ltd. |
| HindIII | Thermo Fisher Scientific (China) Co., Ltd. |
| ClonExpress MultiS One Step Cloning Kit | Nanjing Vazyme Biotech Co., Ltd. |
| 50×TAE buffer | Sangon Bioengineering (Shanghai) Co., Ltd. |
| Brain-heart infusion medium (BHI) | Beijing Aoboxing Biotechnology Co., Ltd. |
| Peptone | Beijing Aoboxing Biotechnology Co., Ltd. |
| Yeast extract powder | Beijing Aoboxing Biotechnology Co., Ltd. |
| Sodium chloride | Sangon Bioengineering (Shanghai) Co., Ltd. |
| Ammonium sulfate | Sangon Bioengineering (Shanghai) Co., Ltd. |
| Urea | Sangon Bioengineering (Shanghai) Co., Ltd. |
| Dipotassium hydrogen phosphate | Sangon Bioengineering (Shanghai) Co., Ltd. |
| Potassium dihydrogen phosphate | Sangon Bioengineering (Shanghai) Co., Ltd. |
| Biotin | Sinopharm Chemical Reagent Co., Ltd. |
| Magnesium sulfate | Sinopharm Chemical Reagent Co., Ltd. |
| Corn steep liquor | Roquette |
| Glucose | Sinopharm Chemical Reagent Co., Ltd. |
| MOPS | Sangon Bioengineering (Shanghai) Co., Ltd. |
| Ferrous sulfate | Sinopharm Chemical Reagent Co., Ltd. |
| VB1•HCl | Sinopharm Chemical Reagent Co., Ltd. |
| Manganese sulfate | Sinopharm Chemical Reagent Co., Ltd. |
| Calcium pantothenate | Sinopharm Chemical Reagent Co., Ltd. |
| Nicotinamide | Sinopharm Chemical Reagent Co., Ltd. |
| Zinc sulfate | Sinopharm Chemical Reagent Co., Ltd. |
| Copper sulfate | Sinopharm Chemical Reagent Co., Ltd. |
| Sorbitol | Sangon Bioengineering (Shanghai) Co., Ltd. |
| Sucrose | Sinopharm Chemical Reagent Co., Ltd. |

The experimental methods involved in the following examples are as follows:

### The PCR amplification system is as follows:

| Ingredient | Volume (microliter) |
|---|---|
| Sterile deionized water | 29 |
| 5×pfu buffer | 10 |
| 2.5 mM dNTP | 5 |
| 10 µM upstream primer | 2 |
| 10 µM downstream primer | 2 |
| Pfu | 1 |
| Template | 1 (The maximum amount of fusion PCR template added is 2 µl) |
| Total | 50 |

### The PCR amplification procedure is as follows:

| Program | Temperature (°C) | Time (s) | Number of cycles (number) |
|---|---|---|---|
| Predenaturation | 95 | 2 min (10-15 min for colony PCR) | 1 |
| Denaturation | 95 | 20 | 30 |
| Annealing | Tm-5 (usually 55°C) | 20 | |
| Extension | 72 | 30 s/KB (extension time ≥ length of fragment to be amplified) | |
| Final extension | 72 | 5min | 1 |

### Strain modification method:

1. Seamless assembly reaction program: referring to the ClonExpress MultiS One Step Cloning Kit instructions.
2. Transformation method: Referring to Trans1-T1 Phage Resistant Chemically Competent Cell instructions.
3. Preparation of competent cells: referring to C. glutamicum Handbook, Chapter 23.

### Example 1 Construction of plasmids for genome modification of strains

### 1. Construction of the plasmid pK18mobsacB-P_{sod}-lysC^{g1a-T311I} for enhancing expression of aspartate kinase

The upstream homologous arm up was obtained by PCR amplification with P21/P22 primer pair using Corynebacterium glutamicum ATCC 13032 genome as template, the promoter fragment Psod was obtained by PCR amplification with P23/P24 primer pair, lysCg1a-T311I was obtained by PCR amplification with P25/P26 primer pair, and the downstream homologous arm dn was obtained by PCR amplification with P27/P28 primer pair. The up-Psod fragment was obtained by fusion PCR with P21/P24 primer pair using up and Psod as templates. The full-length fragment up-Psod-lysCg1aT311I-dn was obtained by fusion PCR with P21/P28 primer pair using up-Psod, lysCg1aT311I and dn as templates. pK18mobsacB was digested with BamHI/HindIII. The two were assembled using a seamless cloning kit and transformed into Trans1 T1 competent cells to obtain the recombinant plasmid pK18mobsacB-P_{sod}-*lysC*^{g1a-T311I}.

Among them, g1a indicated that the base at position 1 of the start codon of the *lysC* gene (the *lysC* wild-type gene sequence is shown in SEQ ID NO: 1) was mutated from g to a, and T311I indicated that the amino acid at position 311 of the aspartate kinase encoded by the *lysC* gene was mutated from T to I.

### 2. Construction of plasmid pK18mobsacB-P_{cspB}-hom^{G378E} for enhanced expression of homoserine dehydrogenase

The upstream homologous arm up was obtained by PCR amplification with P29/P30 primer pair using Corynebacterium glutamicum ATCC 13032 genome as template, the promoter fragment PcspB was obtained by PCR amplification with P31/P32 primer pair using ATCC14067 genome as template, homG378E was obtained by PCR amplification with P33/P34 primer pair using ATCC13032 genome as template, and the downstream homologous arm dn was obtained by PCR amplification with P35/P36 primer pair. The fragment up-PcspB was obtained by fusion PCR with P29/P32 primer pair using up and PcspB as templates. The full length fragment up-PcspB-homG378E-dn was obtained by fusion PCR with P29/P36 primer pair using up-PcspB, homG378E and dn as templates. pK18mobsacB was digested with BamHI/HindIII. The two were assembled using a seamless cloning kit and transformed into Trans1 T1 competent cells to obtain the recombinant plasmid pK18mobsacB-P_{cspB}-*hom*^{G378E}.

### 3. Construction of the plasmid pK18mobsacB-P_{sod}-aspB for enhancing expression of aspartate aminotransferase

The upstream homologous arm up was obtained by PCR amplification with P103/P104 primer pair using Corynebacterium glutamicum ATCC 13032 genome as template, the promoter fragment Psod was obtained by PCR amplification with P105/P106 primer pair, and the downstream homologous arm dn was obtained by PCR amplification with P107/P108 primer pair. The full length fragment up-Psod-dn was obtained by fusion PCR with P103/P108 primer pair using up, Psod and dn as templates. pK18mobsacB was digested with BamHI/HindIII. The two were assembled using a seamless cloning kit and transformed into Trans1 T1 competent cells to obtain the recombinant plasmid pK18mobsacB-P_{sod}-*aspB*.

### 4. Construction of the plasmid pK18mobsacB-P_{sod}-thrC^{g1a} for enhancing expression of threonine synthase

The upstream homologous arm up was obtained by PCR amplification with P37/P38 primer pair using ATCC13032 genome as template, the promoter fragment Psod-thrCg1a was obtained by PCR amplification with P39/P40 primer pair, and the downstream homologous arm dn was obtained by PCR amplification with P41/P42 primer pair. The full length fragment up-Psod-thrCg1a-dn was obtained by fusion PCR with P37/P42 primer pair using up, Psod-thrCV1M, and dn as templates. pK18mobsacB was digested with BamHI/HindIII. The two were assembled using a seamless cloning kit and transformed into Trans1 T1 competent cells to obtain the recombinant plasmid pK18mobsacB-P_{sod}-*thrC*^{g1a}.

Among them, g1a indicated that the base at position 1 of the start codon of the *thrC* gene (the *thrC* wild-type gene sequence is shown in SEQ ID NO: 2) was mutated from g to a.

### 5. Construction of plasmid pK18mobsacB-ΔprpC1 for inactivating 2-methylcitrate synthase 1

The upstream homologous arm up was obtained by PCR amplification with prpC1-UF/ prpC1-UR primer pair using Corynebacterium glutamicum ATCC 13032 genome as template, and the downstream homologous arm dn was obtained by PCR amplification with prpC1-DF/prpC1-DR primer pair. The full length fragment up-dn was obtained by fusion PCR with prpC1-UF/prpC1-DR primer pair using up and dn as templates. pK18mobsacB was digested with BamHI/HindIII. The two were assembled using a seamless cloning kit and transformed into Trans1 T1 competent cells to obtain the recombinant plasmid pK18mobsacB-Δ*prpC1*.

### 6. Construction of plasmid pK18mobsacB-P_{tuf}-ppnK for enhancing expression of NAD kinase

The upstream homologous arm up was obtained by PCR amplification with P109/P110 primer pair using Corynebacterium glutamicum ATCC 13032 genome as template, the promoter fragment Ptuf was obtained by PCR amplification with P111/P112 primer pair, and the downstream homologous arm dn was obtained by PCR amplification with P113/P114 primer pair. The full length fragment up-Ptuf-dn was obtained by fusion PCR with P109/P114 primer pair using up, Ptuf, and dn as templates. pK18mobsacB was digested with BamHI/HindIII. The two were assembled using a seamless cloning kit and transformed into Trans1 T1 competent cells to obtain the recombinant plasmid pK18mobsacB-P_{tuf}-*ppnK*.

### 7. Construction of plasmid pK18mobsacB-P_{tuf}-fbp for enhancing expression of fructose-1,6-bisphosphatase

The upstream homologous arm up was obtained by PCR amplification with P61/P62 primer pair using Corynebacterium glutamicum ATCC 13032 genome as template, the promoter fragment Ptuf was obtained by PCR amplification with P63/P64 primer pair, and the downstream homologous arm dn was obtained by PCR amplification with P65/P66 primer pair. The full length fragment up-Ptuf-dn was obtained by fusion PCR with P61/P66 primer pair using up, Ptuf, and dn as templates. pK18mobsacB was digested with BamHI/HindIII. The two were assembled using a seamless cloning kit and transformed into Trans1 T1 competent cells to obtain the recombinant plasmid pK18mobsacB-P_{tuf}-*fbp*.

The primers used in the construction process are shown in Table 1:

**Table 1**

| Name | Sequence (5'-3') (SEQ ID NO: 3-46 in order) |
|---|---|
| P21 | AATTCGAGCTCGGTACCCGGGGATCCAGCGACAGGACAAGCACTGG |
| P22 | CCCGGAATAATTGGCAGCTATGTGCACCTTTCGATCTACG |
| P23 | CGTAGATCGAAAGGTGCACATAGCTGCCAATTATTCCGGG |
| P24 | TTTCTGTACGACCAGGGCCA**T**GGGTAAAAAATCCTTTCGTA |
| P25 | TACGAAAGGATTTTTTACCC**A**TGGCCCTGGTCGTACAGAAA |
| P26 | TCGGAACGAGGGCAGGTGAAGGTGATGTCGGTGGTGCCGTCT |
| P27 | AGACGGCACCACCGACATCACCTTCACCTGCCCTCGTTCCGA |
| P28 | GTAAAACGACGGCCAGTGCCAAGCTTAGCCTGGTAAGAGGAAACGT |
| P29 | AATTCGAGCTCGGTACCCGGGGATCCCTGCGGGCAGATCCTTTTGA |
| P30 | ATTTCTTTATAAACGCAGGTCATATCTACCAAAACTACGC |
| P31 | GCGTAGTTTTGGTAGATATGACCTGCGTTTATAAAGAAAT |
| P32 | GTATATCTCCTTCTGCAGGAATAGGTATCGAAAGACGAAA |
| P33 | TTTCGTCTTTCGATACCTATTCCTGCAGAAGGAGATATAC |
| P34 | TAGCCAATTCAGCCAAAACCC**C**CACGCGATCTTCCACATCC |
| P35 | GGATGTGGAAGATCGCGTGG**G**GGTTTTGGCTGAATTGGCTA |
| P36 | GTAAAACGACGGCCAGTGCCAAGCTTGCTGGCTCTTGCCGTCGATA |
| P37 | ATTCGAGCTCGGTACCCGGGGATCCGCCGTTGATCATTGTTCTTCA |
| P38 | CCCGGAATAATTGGCAGCTAGGATATAACCCTATCCCAAG |
| P39 | CTTGGGATAGGGTTATATCCTAGCTGCCAATTATTCCGGG |
| P40 | ACGCGTCGAAATGTAGTCCA**T**GGGTAAAAAATCCTTTCGTA |
| P41 | TACGAAAGGATTTTTTACCC**A**TGGACTACATTTCGACGCGT |
| P42 | GTAAAACGACGGCCAGTGCCAAGCTTGAATACGCGGATTCCCTCGC |
| P61 | AATTCGAGCTCGGTACCCGGGGATCCTCATCTGCGGTGACATATCC |
| P62 | CATTCGCAGGGTAACGGCCACTGAAGGGCCTCCTGGGGCA |
| P63 | TGCCCCAGGAGGCCCTTCAGTGGCCGTTACCCTGCGAATG |
| P64 | TCGGGGTTCTTTAGGTTCATTGTATGTCCTCCTGGACTTC |
| P65 | GAAGTCCAGGAGGACATACAATGAACCTAAAGAACCCCGA |
| P66 | GTAAAACGACGGCCAGTGCCAAGCTTGTGACGTCGGAAGGGTTGAT |
| P103 | GAGCTCGGTACCCGGGGATCCGCAGGGTATTGCAGGGACTCA |
| P104 | CAAGCCCGGAATAATTGGCAGCTAAACTGCGTACCTCCGCATGTGGTGG |
| P105 | TAGCTGCCAATTATTCCGGGCTTGT |
| P106 | GGGTAAAAAATCCTTTCGTAGGTTT |
| P107 | GGAAACCTACGAAAGGATTTTTTACCCATGAGTTCAGTTTCGCTGCAGGATTT |
| P108 | ACGACGGCCAGTGCCAAGCTTACACCGGAACAACCCACATG |
| P109 | GAGCTCGGTACCCGGGGATCCGAAGCGTCTGAAGTAGTGGCAGT |
| P110 | ACATTCGCAGGGTAACGGCCATTATTGCGGACCTTCCTTTACAGC |
| P111 | TGGCCGTTACCCTGCGAATGTCCAC |
| P112 | TGTATGTCCTCCTGGACTTCGTGG |
| P113 | CACCACGAAGTCCAGGAGGACATACAATGACTGCACCCACGAACGCTGGGGA |
| P114 | ACGACGGCCAGTGCCAAGCTTGCATCGAGCACTCCCCTGC |
| prpC1-UF | AATTCGAGCTCGGTACCCGGGGATCCACGTGATGGTTCGACGCATC |
| prpC1-UR | AAATCAGCCTCACTGATTAGTCACTCATTGTTTTCTCCTT |
| prpC1-DF | AAGGAGAAAACAATGAGTGACTAATCAGTGAGGCTGATTT |
| prpC1-DR | GTAAAACGACGGCCAGTGCCAAGCTTTGGTTGTCGGATCT |

| | |
|---|---|
| Note: The bold and underlined characters are primers for introducing corresponding point mutations. | |

### Example 2 Construction of a genome-modified strain

### 1. Construction of strain for inactivating 2-methylcitrate synthase 1

Corynebacterium glutamicum ATCC 13032 competent cells were prepared according to the classic method of Corynebacterium glutamicum (C. glutamicum Handbook, Charpter 23). The competent cells were transformed with the recombinant plasmid pK18mobsacB-Δ*prpC1* by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strain of interest was named SMCT089.

### 2. Construction of a strain with enhanced expression of aspartate kinase

ATCC13032 competent cells were prepared according to the classic method of Corynebacterium glutamicum (C. glutamicum Handbook, Charpter 23). The competent cells were transformed with the recombinant plasmid pK18mobsacB-P_{sod}-*lysC*^{g1a-T311I} by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strain of interest was named SMCT076.

### 3. Construction of a strain with enhanced expression of homoserine dehydrogenase

SMCT076 competent cells were prepared according to the classic method of Corynebacterium glutamicum (C. glutamicum Handbook, Charpter 23). The competent cells were transformed with the recombinant plasmid pK18mobsacB-P_{cspB}-*hom*^{G378E} by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strain of interest was named SMCT077.

### 4. Construction of a strain with enhanced expression of threonine synthase

SMCT077 competent cells were prepared according to the classic method of Corynebacterium glutamicum (C. glutamicum Handbook, Charpter 23). The competent cells were transformed with the recombinant plasmid pK18mobsacB-P_{sod}-*thrC*^{g1a} by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strain of interest was named SMCT078.

### 5. Construction of a strain with enhanced expression of NAD kinase

SMCT077 competent cells were prepared according to the classic method of Corynebacterium glutamicum (C. glutamicum Handbook, Charpter 23). The competent cells were transformed with the recombinant plasmid pK18mobsacB-P_{tuf}-*ppnK* by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strain of interest was named SMCT079.

### 6. Construction of a strain with enhanced expression of fructose-1,6-bisphosphatase

SMCT077 and SMCT079 competent cells were prepared according to the classic method of Corynebacterium glutamicum (C. glutamicum Handbook, Charpter 23). The competent cells were transformed with the recombinant plasmid pK18mobsacB-P_{tuf}-*fbp* by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strains of interest were named SMCT080 and SMCT081.

### 7. Modification for enhancing expression of aspartate aminotransferase and threonine synthase in SMCT081

SMCT081 competent cells were prepared according to the classic method of Corynebacterium glutamicum (C. glutamicum Handbook, Charpter 23). The competent cells were transformed with the recombinant plasmid pK18mobsacB-P_{sod}-*aspB* by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing to obtain the mutant strain of interest for further preparation of competent cells. The competent cells were transformed with the recombinant plasmid pK18mobsacB-P_{sod}-*thrC*^{g1a} by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence pf interest was amplified by PCR and analyzed by nucleotide sequencing to obtain the mutant strain of interest SMCT082.

### 8. Construction of strain for inactivating 2-methylcitrate synthase 1

SMCT077, SMCT078, SMCT079, SMCT080, SMCT081, and SMCT082 competent cells were prepared according to the classic method of Corynebacterium glutamicum (C. glutamicum Handbook, Charpter 23). The competent cells were transformed with the recombinant plasmid pK18mobsacB-Δ*prpC1* by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strains of interest were named SMCT083, SMCT084, SMCT085, SMCT086, SMCT087, and SMCT088.

The strains obtained are shown in Table 2:

**Table 2**

| Strains | Genotype |
|---|---|
| SMCT089 | ATCC13032, Δ*prpC1* |
| SMCT076 | ATCC13032, P_{sod}-*lysC*^{g1a-T311I} |
| SMCT077 | ATCC13032, P_{sod}-*lysC*^{g1a-T311I}, P_{cspB}-*hom*^{G378E} |
| SMCT078 | ATCC13032, P_{sod}-*lysC*^{g1a-T311I}, P_{cspB}-*hom*^{G378E}, P_{sod}-*thrC*^{g1a} |
| SMCT079 | ATCC13032, P_{sod}-*lysC*^{g1a-T311I}, P_{cspB}-*hom*^{G378E}, P_{tuf}-*ppnK* |
| SMCT080 | ATCC13032, P_{sod}-*lysC*^{g1a-T311I} P_{cspB}-*hom*^{G378E}, P_{tuf}-*fbp* |
| SMCT081 | ATCC13032, P_{sod}-*lysC*^{g1a-T311I}, P_{cspB}-*hom*^{G378E}, P_{tuf}-*ppnK,* P_{tuf}-*fbp* |
| SMCT082 | ATCC13032, P_{sod}-*lysC*^{g1a-T311I}, P_{cspB}-*hom*^{G378E}, P_{tuf}-*ppnK*, P_{tuf}-*fbp*, P_{sod}*-aspB,* P_{sod}-*thrC*^{g1a} |
| SMCT083 | ATCC13032, P_{sod}-*lysC*^{g1a-T311I}, P_{cspB}-*hom*^{G378E}, Δ*prpC1* |
| SMCT084 | ATCC13032, P_{sod}-*lysC*^{g1a-T311I}, P_{cspB}-*hom*^{G378E}, *P*_{sod}-*thrC*^{g1a}, Δ*prpC1* |
| SMCT085 | ATCC13032, P_{sod}-*lysC*^{g1a-T311I}, P_{cspB}-*hom*^{G378E}, P_{tuf}*-ppnK*, Δ*prpC1* |
| SMCT086 | ATCC13032, P_{sod}-*lysC*^{g1a-T311I}, P_{cspB}-*hom*^{G378E}, P_{tuf}-*fbp*, Δ*prpC1* |
| SMCT087 | ATCC13032, P_{sod}-*lysC*^{g1a,T311I}, P_{cspB}-*hom*^{G378E}, P_{tuf}-*ppnK*, P_{tuf}-*fbp*, Δ*prpC1* |
| SMCT088 | ATCC13032, P_{sod}-*lysC*^{g1a-T311I}, P_{cspB}-*hom*^{G378E}, P_{tuf}-*ppnK*, P_{tuf}*-fbp*, P_{sod}-*aspB*, P*_{sod}-thrC*^{g1a}, Δ*prpC1* |

### Example 3 Shake flask verification of constructed strains

### 1 . Medium

Seed activation medium: BHI 3.7%, agar 2%, pH 7.

Seed medium: Peptone 5/L, yeast extract 5 g/L, sodium chloride 10 g/L, ammonium sulfate 16 g/L, urea 8 g/L, potassium dihydrogen phosphate 10.4 g/L, dipotassium hydrogen phosphate 21.4 g/L, biotin 5 mg/L, magnesium sulfate 3 g/L. Glucose 50 g/L, pH 7.2.

Fermentation medium: corn steep liquor 50 mL/L, glucose 30 g/L, ammonium sulfate 4 g/L, MOPS 30 g/L, potassium dihydrogen phosphate 10 g/L, urea 20 g/L, biotin 10 mg/L, magnesium sulfate 6 g/L, ferrous sulfate 1 g/L, VB1•HCl 40 mg/L, calcium pantothenate 50 mg/L, nicotinamide 40 mg/L, manganese sulfate 1 g/L, zinc sulfate 20 mg/L, copper sulfate 20 mg/L, pH 7.2.

### 2 . Production of L-threonine by shake flask fermentation with engineered strain

(1) Seed culture: 1 loop of seed of SMCT076, SMCT077, SMCT078, SMCT079, SMCT080, SMCT081, SMCT082, SMCT083, SMCT084, SMCT085, SMCT086, and SMCT088 on the slant culture medium was picked and inoculated into a 500 mL Erlenmeyer flask containing 20 mL of seed culture medium, and cultured at 30°C and 220 r/min for 16 h.
(2) Fermentation culture: 2 mL of seed liquid was inoculated into a 500 mL Erlenmeyer flask containing 20 mL of fermentation medium and cultured at 33°C and 220 r/min under shaking for 24 h.
(3) 1 mL of fermentation broth was taken and centrifuged (12000 rpm, 2 min), and the supernatant was collected. L-threonine in the fermentation broth of engineered strain and control strain were detected by HPLC.

The comparison of threonine-producing ability of Corynebacterium glutamicum is shown in Table 3:

**Table 3**

| Strain number | OD₅₆₂ | Threonine (g/L) | Strain number | OD₅₆₂ | Threonine (g/L) |
|---|---|---|---|---|---|
| ATCC13032 | 25 | - | SMCT089 | 25 | 0.2 |
| SMCT076 | 23 | 1.2 | - | - | - |
| SMCT077 | 23 | 2.4 | SMCT083 | 23 | 3.3 |
| SMCT078 | 24 | 3.0 | SMCT084 | 24 | 4.1 |
| SMCT079 | 24 | 3.3 | SMCT085 | 24 | 4.5 |
| SMCT080 | 23 | 3.5 | SMCT086 | 23 | 5.0 |
| SMCT081 | 22 | 8.0 | SMCT087 | 22 | 9.2 |
| SMCT082 | 22 | 10.2 | SMCT088 | 22 | 12.5 |

As can be seen from Table 3, after the aspartate kinase was modified on the basis of the wild strain ATCC13032, the threonine production of the strain was initially accumulated. With the enhanced expression of enzymes (Homoserine dehydrogenase, aspartate aminotransferase, and threonine synthase) in the threonine synthesis pathway, the threonine production was further improved. Subsequently, the expression of NAD kinase and fructose 1,6-bisphosphatase was enhanced, and the reducing power supply of the strain was further strengthened, which was conducive to the improvement of threonine production.

In order to explore the effect of 2-methylcitrate synthase 1 on threonine production, 2-methylcitrate synthase 1 was further inactivated on the basis of threonine-producing bacteria. It can be seen from Table 3 that the threonine production of all modified bacteria for 2-methylcitrate synthase 1 was improved to varying degrees, with the highest improvement by 42% compared to the control strain. It can be seen that the inactivation of 2-methylcitrate synthase 1 was conducive to the improvement of threonine production of the strain.

The above are only preferred embodiments of the present invention. It should be noted that those skilled in the art can make some improvements and modifications without departing from the principles of the present invention, for example, expression enhancement can be achieved by using a strong promoter, changes of the RBS sequence, changes of the start codon, changes of the amino acid sequence to enhance activity, etc, and expression inactivation includes inactivation and attenuation of protein activity, etc. These improvements and modifications are all within the scope of protection claimed by the present invention.

### Description of sequences

SEQ ID No: 1 Corynebacterium glutamicum lysC wild-type gene
gtggccctgg tcgtacagaa atatggcggt tcctcgcttg agagtgcgga acgcattaga 60
aacgtcgctg aacggatcgt tgccaccaag aaggctggaa atgatgtcgt ggttgtctgc 120
tccgcaatgg gagacaccac ggatgaactt ctagaacttg cagcggcagt gaatcccgtt 180
ccgccagctc gtgaaatgga tatgctcctg actgctggtg agcgtatttc taacgctctc 240
gtcgccatgg ctattgagtc ccttggcgca gaagcccaat ctttcacggg ctctcaggct 300
ggtgtgctca ccaccgagcg ccacggaaac gcacgcattg ttgatgtcac tccaggtcgt 360
gtgcgtgaag cactcgatga gggcaagatc tgcattgttg ctggtttcca gggtgttaat 420
aaagaaaccc gcgatgtcac cacgttgggt cgtggtggtt ctgacaccac tgcagttgcg 480
ttggcagctg ctttgaacgc tgatgtgtgt gagatttact cggacgttga cggtgtgtat 540
accgctgacc cgcgcatcgt tcctaatgca cagaagctgg aaaagctcag cttcgaagaa 600
atgctggaac ttgctgctgt tggctccaag attttggtgc tgcgcagtgt tgaatacgct 660
cgtgcattca atgtgccact tcgcgtacgc tcgtcttata gtaatgatcc cggcactttg 720
attgccggct ctatggagga tattcctgtg gaagaagcag tccttaccgg tgtcgcaacc 780

gacaagtccg aagccaaagt aaccgttctg ggtatttccg ataagccagg cgaggctgcg 840
aaggttttcc gtgcgttggc tgatgcagaa atcaacattg acatggttct gcagaacgtc 900
tcttctgtag aagacggcac caccgacatc accttcacct gccctcgttc cgacggccgc 960
cgcgcgatgg agatcttgaa gaagcttcag gttcagggca actggaccaa tgtgctttac 1020
gacgaccagg tcggcaaagt ctccctcgtg ggtgctggca tgaagtctca cccaggtgtt 1080
accgcagagt tcatggaagc tctgcgcgat gtcaacgtga acatcgaatt gatttccacc 1140
tctgagattc gtatttccgt gctgatccgt gaagatgatc tggatgctgc tgcacgtgca 1200
ttgcatgagc agttccagct gggcggcgaa gacgaagccg tcgtttatgc aggcaccgga 1260
cgctaa 1266
SEQ ID No: 2 Corynebacterium glutamicum thrC wild-type gene
gtggactaca tttcgacgcg tgatgccagc cgtacccctg cccgcttcag tgatattttg 60
ctgggcggtc tagcaccaga cggcggcctg tacctgcctg caacctaccc tcaactagat 120
gatgcccagc tgagtaaatg gcgtgaggta ttagccaacg aaggatacgc agctttggct 180
gctgaagtta tctccctgtt tgttgatgac atcccagtag aagacatcaa ggcgatcacc 240
gcacgcgcct acacctaccc gaagttcaac agcgaagaca tcgttcctgt caccgaactc 300
gaggacaaca tttacctggg ccacctttcc gaaggcccaa ccgctgcatt caaagacatg 360
gccatgcagc tgctcggcga acttttcgaa tacgagcttc gccgccgcaa cgaaaccatc 420
aacatcctgg gcgctacctc tggcgatacc ggctcctctg cggaatacgc catgcgcggc 480
cgcgagggaa tccgcgtatt catgctgacc ccagctggcc gcatgacccc attccagcaa 540
gcacagatgt ttggccttga cgatccaaac atcttcaaca tcgccctcga cggcgttttc 600
gacgattgcc aagacgtagt caaggctgtc tccgccgacg cagaattcaa aaaagacaac 660
cgcatcggtg ccgtgaactc catcaactgg gcacgcctta tggcacaggt tgtgtactac 720
gtttcctcat ggatccgcac cacaaccagc aatgaccaaa aggtcagctt ctccgtacca 780
accggcaact tcggtgacat ttgcgcaggc cacatcgccc gccaaatggg acttcccatc 840
gatcgcctca tcgtggccac caacgaaaac gatgtgctcg acgagttctt ccgtaccggc 900
gactaccgag tccgcagctc cgcagacacc cacgagacct cctcaccttc gatggatatc 960
tcccgcgcct ccaacttcga gcgtttcatc ttcgacctgc tcggccgcga cgccacccgc 1020
gtcaacgatc tatttggtac ccaggttcgc caaggcggat tctcactggc tgatgacgcc 1080
aactttgaga aggctgcagc agaatacggt ttcgcctccg gacgatccac ccatgctgac 1140
cgtgtggcaa ccatcgctga cgtgcattcc cgcctcgacg tactaatcga tccccacacc 1200
gccgacggcg ttcacgtggc acgccagtgg agggacgagg tcaacacccc aatcatcgtc 1260
ctagaaactg cactcccagt gaaatttgcc gacaccatcg tcgaagcaat tggtgaagca 1320
cctcaaactc cagagcgttt cgccgcgatc atggatgctc cattcaaggt ttccgaccta 1380
ccaaacgaca ccgatgcagt taagcagtac atagtcgatg cgattgcaaa cacttccgtg 1440
aagtaa 1446

## Claims

1. A modified microorganism from the genus *Corynebacterium,* wherein the modified microorganism has its 2-methylcitrate synthase 1 activity reduced or lost as compared to the unmodified microorganism, and has improved threonine production as compared to the unmodified microorganism.

2. The modified microorganism of claim 1, wherein the 2-methylcitrate synthase 1 activity in the modified microorganism is reduced or lost by reducing the expression of a gene encoding 2-methylcitrate synthase 1 or by knocking out an endogenous gene encoding 2-methylcitrate synthase 1.

3. The modified microorganism of claim 2, wherein the reducing the expression of a gene encoding 2-methylcitrate synthase 1 or the knocking out an endogenous gene encoding 2-methylcitrate synthase 1 is performed by mutagenesis, site-directed mutation, or homologous recombination.

4. The modified microorganism of claim 1, wherein the modified microorganism has enhanced activity of an enzyme involved in the *in vivo* threonine synthesis pathway compared to the unmodified microorganism;
wherein the enzyme involved in the threonine synthesis pathway is at least one selected from aspartate aminotransferase, aspartate kinase, homoserine dehydrogenase, threonine synthase, NAD kinase, and fructose-1,6-bisphosphatase 2.

5. The modified microorganism of claim 4, wherein the modified microorganism is any one of the following (1) to (5):
(1) a microorganism with reduced or lost activity of 2-methylcitrate synthase 1 and enhanced activity of aspartate kinase and/or homoserine dehydrogenase;
(2) a microorganism with reduced or lost activity of 2-methylcitrate synthase 1 and enhanced activity of aspartate kinase, homoserine dehydrogenase, and/or threonine synthase;
(3) a microorganism with reduced or lost activity of 2-methylcitrate synthase 1 and enhanced activity of aspartate kinase, homoserine dehydrogenase, and/or NAD kinase;
(4) a microorganism with reduced or lost activity of 2-methylcitrate synthase 1 and enhanced activity of aspartate kinase, homoserine dehydrogenase, NAD kinase, and/or fructose-1,6-bisphosphatase 2; and
(5) a microorganism with reduced or lost activity of 2-methylcitrate synthase 1 and enhanced activity of aspartate aminotransferase, aspartate kinase, homoserine dehydrogenase, threonine synthase, NAD kinase, and fructose-1,6-bisphosphatase 2.

6. The modified microorganism of claim 4, wherein the activity of the enzyme involved in the *in vivo* threonine synthesis pathway in the modified microorganism is enhanced by any one of or any combination of the following 1) to 6):
1) introducing a plasmid carrying the gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme; and
6) altering the nucleotide sequence encoding the enzyme.

7. The modified microorganism of any one of claims 1 to 6, which is *Corynebacterium glutamicum.*

8. A method for constructing a threonine-producing engineered bacterium, comprising:
A) attenuating a gene encoding 2-methylcitrate synthase 1 in *Corynebacterium* species, which has an amino acid-producing ability, to obtain an attenuated strain, wherein the attenuating includes knocking out or reducing the expression of the gene encoding 2-methylcitrate synthase 1; and optionally
B) enhancing an enzyme involved in the threonine synthesis pathway in the attenuated strain obtained by step A, to obtain a strain with enhanced enzyme activity; wherein the enhancing is achieved by any one of or any combination of the following 1) to 5):
1) introducing a plasmid carrying the gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme;
wherein the enzyme involved in the threonine synthesis pathway is at least one selected from aspartate aminotransferase, aspartate kinase, homoserine dehydrogenase, threonine synthase, NAD kinase, and fructose-1,6-bisphosphatase 2.

9. The method of claim 8, wherein the *Corynebacterium* species is *Corynebacterium glutamicum.*

10. A method for producing threonine, comprising the following steps:
a) culturing the modified microorganism of any one of claims 1-7 or the engineered bacterium constructed by the method of claim 8 or 9 to obtain a culture of the modified microorganism or the engineered bacterium;
b) collecting threonine from the culture obtained in step a).
